# EUROPÄISCHE PATENTANMELDUNG

(11) **EP 4 497 452 A1**
(43) Veröffentlichungstag der Anmeldung: **29.01.2025**
(21) Anmeldenummer: 24190362.4
(22) Anmeldetag: 23.07.2024
(51) Int. Cl.: A61M 1/16

(54) **ANSAUGEINHEIT FÜR EIN MEDIZINISCHES GERÄT**

(30) Priorität: 24.07.2023 DE 102023119498
(71) Anmelder: B. Braun Avitum AG, 34212 Melsungen (DE)
(72) Erfinder: JANIK, Waldemar, 34212 Melsungen (DE)
(74) Vertreter: Winter, Brandl - Partnerschaft mbB

(57) **Zusammenfassung**

Offenbart sind eine Ansaugeinheit (6) und ein medizinisches Gerät (100) mit einer Ansaugeinheit und eine medizinische Anordnung mit einer Ansaugeinheit (6), wobei die Ansaugeinheit (6) zur fluidischen Verbindung mit einem Behälter, vorzugsweise Kanister (10), vorbereitet und ausgelegt und eingerichtet ist, oder wobei die Ansaugeinheit (6) an einem Behälter, vorzugsweise Kanister (10), befestigt ist. Die Ansaugeinheit (6) weist einen äußeren Anschlussstutzen (3) auf, über den sie mit dem medizinischen Gerät (100), vorzugsweise einer Dialysemaschine mechanisch und fluidisch verbindbar oder lösbar verbunden ist.

## Beschreibung

### Technisches Gebiet

Die vorliegende Offenbarung betrifft eine Ansaugeinheit für ein medizinisches Gerät, insbesondere für eine Dialysemaschine um eine Flüssigkeit, insbesondere ein Dialysekonzentrat aus einem Behälter, vorzugsweise einem Kanister, zu saugen und einem medizinischen Prozess, insbesondere einem Dialyseprozess, zuzuführen.

### Hintergrund der Offenbarung

Mit Dialysemaschinen wird das Blut eines Patienten gereinigt, wenn z.B. dessen Nierenfunktion eingeschränkt oder eingestellt ist. Dazu hat die Dialysemaschine einen Dialysator, der einerseits von dem zu reinigenden Blut des Patienten und andererseits von einer Dialysierflüssigkeit durchströmt wird, wobei gewisse gelöste Substanzen (z.B. Harnstoff) aus dem Blut an die Dialysierflüssigkeit übergehen.

Dafür ist es nötig, dass der Dialysemaschine Konzentrate zugeführt werden.

Für pulverförmige bzw. kristalline Konzentrate können an der Dialysemaschine speziell vorgesehene Kartuschen oder Beutel angebracht werden. Hierfür werden zwar weder Ansaugrohre noch Köcher benötigt. Allerdings werden insbesondere saure Konzentrate aufgrund ihrer Zusammensetzung nur in wässriger Lösung bereitgestellt.

### Stand der Technik

Die DE 88 13 659 U1 beschreibt einen Beutel, der mit seinem oberen Ende hängend an einem Traggestell der Dialysemaschine befestigt und am unteren Ende über einen Konnektor mit einem Einstechdorn an einem Entnahmeschlauch angeschlossen ist. Im Gegensatz zu starren Kanistern haben befüllte Beutel allerdings den Nachteil, dass sie schlechter stapelbar und transportierbar sind.

Zum Ansaugen von wässrigen Dialysekonzentraten sind Dialysemaschinen mit Ansaugrohren versehen, die in (externe wechselbare) Kanister gesteckt werden. Um die Ansaugrohre z.B. nach einer Dialysetherapie spülen und reinigen zu können, werden sie in Köcher gesteckt, die Bestandteil der Dialysemaschine sind. Der Nachteil der Köcher ist, dass sie recht viel Platz im Inneren der Dialysemaschine beanspruchen.

### Kurzbeschreibung der Offenbarung

Aufgabe der vorliegenden Offenbarung ist es, den durch den Köcher benötigten Platz bzw. Bauraum im Innern vom medizinischen Geräten, insbesondere von Dialysemaschinen freizugeben.

Diese Aufgabe wird gelöst mit einer Ansaugeinheit für ein medizinisches Gerät mit der Merkmalskombination des Anspruchs 1 und durch ein medizinisches Gerät mit der Merkmalskombination des Anspruchs 6 und durch eine medizinische Anordnung mit der Merkmalskombination des Anspruchs 11.

Die Ansaugeinheit gemäß der Offenbarung ist zur mechanischen und fluidischen Verbindung mit einem (starren) Behälter, vorzugsweise einem Kanister, vorbereitet und ausgelegt und eingerichtet, oder die Ansaugeinheit ist an einem (starren) Behälter, vorzugsweise einem Kanister, befestigt oder gebildet. Die Ansaugeinheit weist einen äußeren Anschlussstutzen auf, über den sie mit dem medizinischen Gerät, mechanisch und fluidisch verbindbar (koppelbar) oder mechanisch und fluidisch lösbar verbunden (gekoppelt) ist. Dieser behälterseitige/ kanisterseitige Anschlussstutzen und seine Koppelmöglichkeit mit dem medizinischen Gerät ermöglicht es, den durch den Köcher benötigten Platz bzw. Bauraum im Innern des medizinischen Gerätes freizugeben, da das medizinische Gerät seinerseits kein Ansaugrohr und entsprechend keine Köcher mehr benötigt.

Wenn die Ansaugeinheit an dem Behälter, vorzugsweise Kanister, befestigt oder (insbesondere einstückig) gebildet ist, kann der Gegenstand als Flüssigkeitsversorgungseinrichtung bezeichnet werden.

Bei einem besonders bevorzugten ersten Prinzip der Offenbarung hat die Ansaugeinheit einen Behälterdeckel, vorzugsweise einen Kanisterdeckel, mittels dem sie an einer Oberseite des Behälters, vorzugsweise Kanisters, anschraubbar ist, wobei an einer Unterseite des Behälterdeckels/ Kanisterdeckels eine Saugleitung angeordnet ist. Diese ragt im Betrieb der Ansaugeinheit in den Behälter/ Kanister hinein, vorzugsweise bis zu einem Boden des Behälters/ Kanisters.

Das erste Prinzip der Offenbarung hat den Vorteil, dass durch die Verlagerung des Ansaugrohres vom medizinischen Gerät zur Ansaugeinheit bzw. in den Behälter/ Kanister im medizinischen Gerät selbst kein Platz mehr für einen Köcher vorgehalten werden muss.

Die Saugleitung kann ein Schlauch sein. Damit ist es bei reichlicher Bemessung des Schlauches möglich, die Flexibilität der Ansaugeinheit bei neuen Behältern/ Kanistern zu erhöhen, die sich ggf. in der Höhe geändert haben. Die Saugleitung kann ein Ansaugrohr (ein Ansaugstab bzw. eine Lanze) sein. Durch das mit dem Behälter/ Kanister bereitgestellte Ansaugrohr können neue Behälter/ Kanister, die sich ggf. in der Höhe geändert haben, leicht integriert werden, ohne hierfür Anpassungen am Köcher oder dem geräteseitigen Ansaugrohr des Standes der Technik vornehmen zu müssen.

Bei einer besonders bevorzugten Weiterbildung des ersten Prinzips ist der äußere Anschlussstutzen ein oberer Anschlussstutzen, der einstückig am Behälterdeckel/ Kanisterdeckel gebildet ist.

Gemäß einem zweiten Prinzip der Offenbarung ist es auch möglich, dass die Ansaugeinheit an einem unteren Wandabschnitt oder an einem Boden des Behälters/ Kanisters befestigt oder (z.B. einstückig) gebildet ist. Dadurch ist weder an dem bauraumkritischen medizinischen Gerät noch im Behälter/ Kanister eine Saugleitung nötigt, wodurch der vorrichtungstechnische Aufwand verringert ist.

Vorzugsweise hat der äußere Anschlussstutzen an seinem Außenumfang eine Hinterschneidung mit mindestens einer Nut. Wenn die Nut umlaufend ausgebildet ist, dann ist beim Aufsetzen einer Kupplung keine Drehposition zu beachten, wodurch das schnelle mechanische Ankoppeln und fluidische Verbinden der Ansaugeinheit und damit des Behälters/ Kanisters an das medizinische Gerät vereinfacht sind.

Die Aufgabe wird auch gelöst durch ein medizinisches Gerät, insbesondere eine Dialysemaschine, die eine geräteseitige Verbindungsleitung aufweist, die einerseits an dem medizinischen Gerät befestigt ist, und an der andererseits die Kupplung befestigt ist. Die Kupplung ist zur lösbaren mechanischen (vorzugsweise formschlüssigen) und fluidischen Verbindung mit dem äußeren Anschlussstutzen der Ansaugeinheit ausgelegt und eingerichtet.

Vorzugsweise sind an einem Innenumfang der Kupplung Rastkörper (z.B. Rastnasen oder insbesondere Rastkugeln) angeordnet, die über eine axial bewegliche (vorzugsweise federvorgespannte) Betätigungshülse radial in die Nut bewegbar sind.

Bei einer bevorzugten Weiterbildung des medizinischen Gerätes ist (z.B. an einer äußeren Wandung, insbesondere an einer vorderen oder seitlichen Wand) ein Stecknippel angeordnet, auf den die Kupplung gesteckt werden kann, wenn dieser vom äußeren Anschlussstutzen getrennt ist, insbesondere während der Behälter/ Kanister gewechselt wird.

Bei einer bevorzugten Weiterbildung des medizinischen Gerätes ist eine Sensorik in Wirkverbindung mit dem Stecknippel vorgesehen, die zum Detektieren der aufgesteckten Kupplung ausgelegt und eingerichtet ist. Dann kann z.B. eine Pumpe des medizinischen Gerätes erst gestartet werden, wenn die Abwesenheit der Kupplung am Stecknippel von der Sensorik gemeldet wird.

Bei einer bevorzugten Weiterbildung des medizinischen Gerätes weist dieses eine Abstellfläche für den Behälter/ Kanister auf. Damit ist eine mechanische Überbelastung der Verbindung zwischen der Kupplung und dem äußeren Anschlussstutzen der Ansaugeinheit z.B. durch versehentliches Verschieben des medizinischen Gerätes bei gleichzeitigem Stehenlassen des Behälters/ Kanisters vermieden.

Die Aufgabe wird auch gelöst durch eine medizinische Anordnung insbesondere Dialyseanordnung mit einem medizinischen Gerät, insbesondere Dialysemaschine gemäß der Offenbarung und mit einem Behälter, vorzugsweise Kanister, wobei das medizinische Gerät und der Behälter/ Kanister mittels einer Ansaugeinheit gemäß der Offenbarung miteinander fluidisch verbindbar oder verbunden sind.

Nachstehend werden Ausführungsbeispiele der vorliegenden Offenbarung auf der Basis der zugehörigen Figuren beschrieben.

Kurzbeschreibung der Figuren
Figur 1 ist ein wesentlicher Teil einer Ansaugeinheit gemäß einem ersten Ausführungsbeispiel der vorliegenden Offenbarung;
Figur 2 ist die gesamte Ansaugeinheit gemäß einem Ausführungsbeispiel der vorliegenden Offenbarung;
Figur 3 ist ein Kanister mit der Ansaugeinheit gemäß dem Ausführungsbeispiel aus Figur 2;
Figur 4 ist eine Dialyseanordnung mit einer Dialysemaschine, an der eine Ansaugeinheit gemäß dem Ausführungsbeispiel aus Figur 2 befestigt ist, und mit einem Kanister;
Figur 5 ist ein Detail der Dialysemaschine aus Figur 4; und
Figur 6 ist das Ausführungsbeispiel der Ansaugeinheit aus Figur 2 mit einem abweichenden Kanister.

### Beschreibung der Ausführungsbeispiele

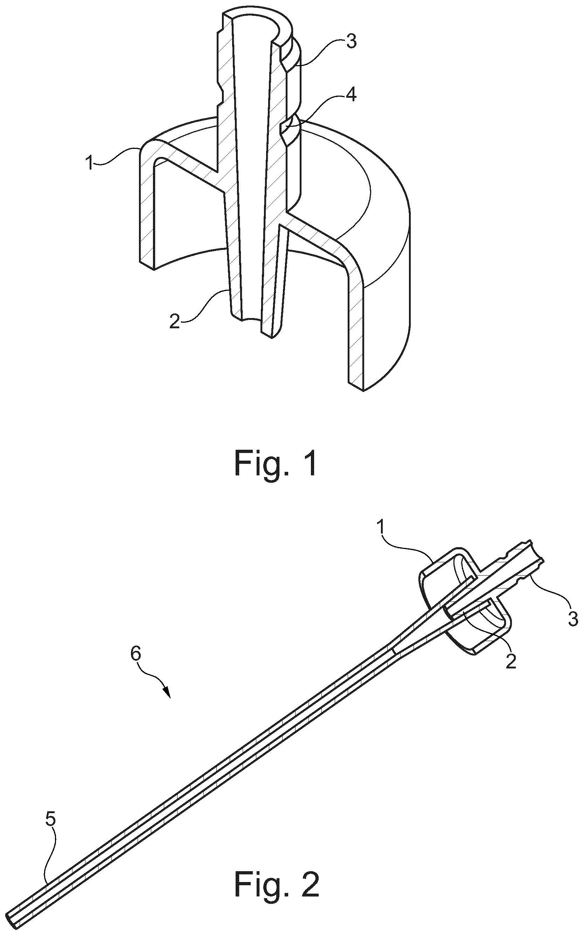

Figur 1 zeigt einen wesentlichen Teil einer Ansaugeinheit 6 gemäß einem Ausführungsbeispiel der vorliegenden Offenbarung.

Figur 1 zeigt schematisch im Halbschnitt einen Kanisterdeckel 1 mit einem unteren Anschlussstutzen 2 und einem oberen Anschlussstutzen 3. Der Kanisterdeckel 1 ist mit dem unteren Anschlussstutzen 2 und dem oberen Anschlussstutzen 3 einstückig gebildet, wobei letzterer eine umlaufende Nut 4 oder eine Hinterschneidung am Außenumfang zum sicheren und reversiblen Fixieren einer Kupplung einer Dialysemaschine aufweist.

Figur 2 ist die gesamte Ansaugeinheit 6 gemäß dem Ausführungsbeispiel der vorliegenden Offenbarung im Halbschnitt.

Am unteren Stutzen 2 des Kanisterdeckels 1 kann eine kanisterseitige Saugleitung 5 in Form eines Schlauches angebracht sein. Die Saugleitung 5 kann auch als starres Ansaugrohr und/oder fester Bestandteil des Kanisterdeckels 1 ausgebildet sein. Der flexible Schlauch hat bei reichlicher Längenbemessung den Vorteil, dass die Ansaugeinheit für Kanister mit verschiedenen Höhen geeignet ist, da der überschüssige Schlauchabschnitt einfach am Boden liegen kann.

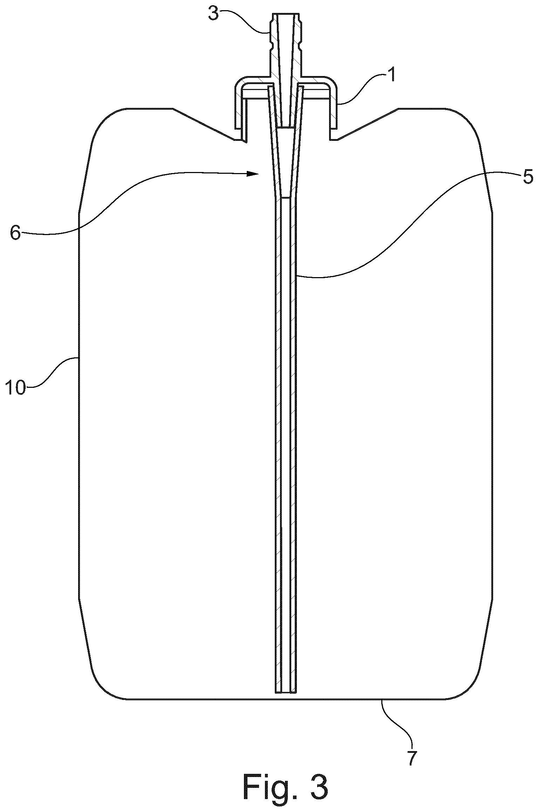

Figur 3 zeigt einen Kanister 10 mit der Ansaugeinheit 6 gemäß dem Ausführungsbeispiel aus Figur 2 im Halbschnitt. Der Schlauch hängt in den Kanister 10 hinein und endet knapp oberhalb des Bodens 7.

Der Kanisterdeckel 1 kann am Kanister 10 mittels eines (nicht gezeigten) Gewindes angeschraubt werden. Alternativ kann der Kanisterdeckel 1 aufgesteckt werden oder aber überstülpbar sein, falls er aus einem teilelastischen Material besteht.

Zur besseren Belüftung beim Entleeren des Kanisters 10 kann im Kanisterdeckel 1 eine Öffnung vorgesehen sein.

Zum Wiederverschließen des Kanisters 10 kann die komplette Ansaugeinheit 6 entnommen und durch einen herkömmlichen allseits geschlossenen Schraubdeckel ersetzt werden. Alternativ kann die Ansaugeinheit 6 am bzw. im Kanister 10 verbleiben, wobei dann eine (nicht gezeigte) Verschlusskappe vorgesehen ist, die dann auf den oberen Stutzen 3 gesteckt wird.

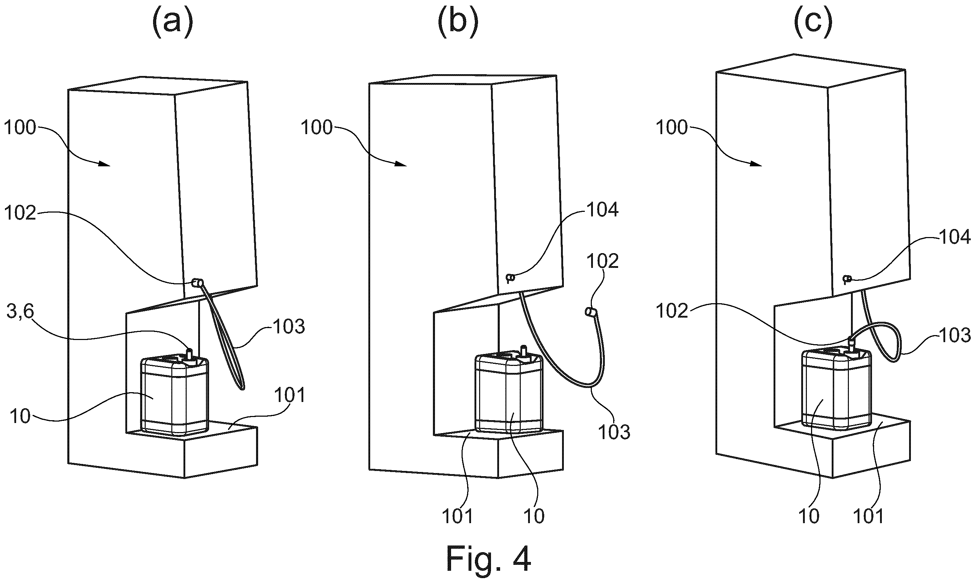

Figur 4 zeigt eine Dialyseanordnung mit einer Dialysemaschine 100. Die Dialysemaschine 100 verfügt über eine Abstellfläche 101, auf die der Kanister 10 gestellt wird. An der Dialysemaschine ist eine geräteseitige Verbindungsleitung 103 angeordnet, die an ihrem freien Endabschnitt eine Kupplung 102 aufweist.

Figur 4 stellt in ihren drei Teilen a b und c drei Zustände der Dialyseanordnung dar. In Figur 4a ist der Kanister 10 nicht an der Dialysemaschine 100 angeschlossen. Die Kupplung 102 ruht auf einem geräteseitigen Stecknippel. In Figur 4b ist die Kupplung 102 bereits vom geräteseitigen Stecknippel 104 entnommen. Figur 4c zeigt die Kupplung 102 aufgesteckt auf dem Kanister 10, genauer gesagt auf den äußeren Anschlussstutzen 3 der Ansaugeinheit 6. Über die Leitung 103 kann die wässrige Lösung aus dem Kanister 10 in das Dialysemaschine 100 gesaugt werden.

Das Dialysemaschine 100 kann über eine (nicht gezeigte) Sensorik zum Detektieren der Kupplung 102 verfügen. Beispielsweise kann in der Kupplung 102 ein Magnet verbaut sein, der einen Reed-Sensor, der sich wiederum in unmittelbarer Nähe des Stecknippels 104 befindet, aktiviert.

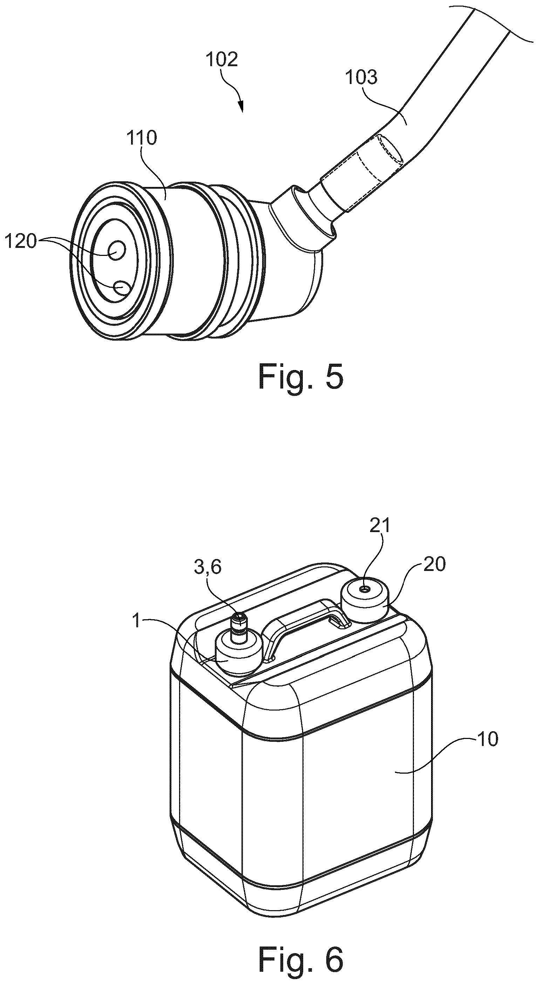

Eine mögliche Ausführung der Kupplung 102 der geräteseitigen Verbindungsleitung 103 ist in Figur 5 zu sehen. Analog zu herkömmlichen Dialysatorkupplungen des Standes der Technik weist die Kupplung 102 eine federgeführte Hülse 110 auf, die zum Diskonnektieren vom Stecknippel 104 oder vom Anschlussstutzen 3 nach hinten geschoben werden muss, um in der Kupplung 102 enthaltene Rastkugeln 120 aus der Hinterschneidung bzw. der Nut 4 zu ziehen. Beim Loslassen der Hülse 110 werden die Rastkugeln 120 wieder in die innere Position gedrückt.

Gemäß einem zweiten Prinzip der vorliegenden Offenbarung ist es auch möglich die Position des Anschlussstutzens 3 ans untere Ende des Kanisters 10 zu verlagern. Auf diese Weise würde eine von unten nach oben verlaufende Saugleitung 5 im Kanister 10 entfallen.

Es ist auch möglich, den Kanister 10 so zu gestalten, dass dieser von Beginn an die Ansaugeinheit 6 gemäß der Offenbarung umfasst. Die Ansaugeinheit 6 wäre demnach kein separates Teil. Der so gebildete Gegenstand wird als Flüssigkeitsversorgungseinrichtung bezeichnet.

Beim ersten Prinzip wäre demnach die Ansaugeinheit 6 mit dem Kanisterdeckel 1 samt der beiden Stutzen 2, 3 und der Saugleitung 5 kein separates Teil. Stattdessen verfügt der Kanister 10 über eine Saugleitung 5 (Schlauch oder Ansaugrohr), die bis zum Boden 7 des Kanisters 10 reicht und oben über die Kupplung 102 konnektiert werden kann. Zum Verschließen des Anschlussstutzens 3 kann dann ein weiterer Verschlussstopfen zum Einsatz kommen (nicht dargestellt).

Zwar wurde in Figur 4 jeweils nur ein Kanister 10 samt zugeordneter Ansaugeinheit 6 zum Ansaugen des Kanisterinhalts dargestellt. Allerdings umfasst die Offenbarung auch die Variante, die Anzahl von Kanistern 10 mit Ansaugeinheiten 6 zu erhöhen. Vorzugsweise umfasst eine Dialysemaschine 100 die Komponenten Kupplung 102, geräteseitige Verbindungsleitung 103 und Stecknippel 104 in doppelter Ausführung, um zwei Konzentrate aus zwei Kanistern 10 ansaugen zu können.

Figur 6 stellt eine weitere mögliche Ausführungsform eines Kanisters 10 dar. Analog zu den obigen Beschreibungen und den Figuren 1, 2, 3 und 4 verfügt der Kanister 10 über eine Ansaugeinheit 6 zum Konnektieren mit einer Kupplung 102. Wie bereits erwähnt, kann am Kanisterdeckel 1 der Ansaugeinheit 6 eine Saugleitung 5 angebracht sein, die in den Kanister 10 hineinragt.

Um auch mit herkömmlichen Dialysemaschinen, die über eigene Ansaugrohre verfügen, Konzentrat aus dem Kanister 10 ansaugen zu können, weist der Kanister 10 zusätzlich eine weitere Anschlussmöglichkeit auf. Dazu ist ein Kanisterdeckel 20 vorgesehen, durch dessen Loch 21 ein aus dem Stand der Technik bekanntes herkömmliches maschinenseitiges Ansaugrohr gesteckt werden kann. In diesem Fall entfällt die Konnektierung über den Anschlussstutzen 3.

### Bezugszeichenliste:

- 1: Kanisterdeckel
- 2: unterer Anschlussstutzen
- 3: äußerer Anschlussstutzen / oberer Anschlussstutzen
- 4: Nut
- 5: Saugleitung
- 6: Ansaugeinheit
- 7: Kanisterboden
- 10: Kanister
- 20: herkömmlicher Kanisterdeckel
- 21: Durchführung
- 100: medizinisches Gerät / Dialysemaschine
- 101: Abstellfläche
- 102: Kupplung
- 103: geräteseitige Verbindungsleitung
- 104: Stecknippel
- 110: Betätigungshülse
- 120: Rastkörper / Rastkugel

## Patentansprüche

1. Ansaugeinheit (6), die zur mechanischen und fluidischen Verbindung mit einem Behälter, vorzugsweise einem Kanister (10), ausgelegt und eingerichtet ist, oder die an einem Behälter, vorzugsweise einem Kanister (10), befestigt ist, **dadurch gekennzeichnet, dass** die Ansaugeinheit (6) einen äußeren Anschlussstutzen (3) aufweist, über den die Ansaugeinheit (6) mit einem medizinischen Gerät (100), vorzugsweise einer Dialysemaschine, mechanisch und fluidisch verbindbar oder lösbar verbunden ist.

2. Ansaugeinheit nach Anspruch 1, **dadurch gekennzeichnet, dass** die Ansaugeinheit (6) einen Behälterdeckel, vorzugsweise Kanisterdeckel (1), aufweist, mittels dem sie an einer Oberseite des Behälters (10) mechanisch und fluidisch verbindbar, vorzugsweise anschraubbar ist, und wobei an einer Unterseite des Behälterdeckels (1) eine Saugleitung (5) angeordnet ist.

3. Ansaugeinheit nach Anspruch 2, **dadurch gekennzeichnet, dass** der äußere Anschlussstutzen ein oberer Anschlussstutzen (3) ist, der einstückig am Behälterdeckel (1) gebildet ist.

4. Ansaugeinheit nach Anspruch 1, **dadurch gekennzeichnet, dass** die Ansaugeinheit (6) an einem unteren Wandabschnitt oder an einem Boden (7) des Behälters (10) angeordnet ist.

5. Ansaugeinheit nach einem der vorhergehenden Ansprüche, **dadurch gekennzeichnet, dass** der äußere Anschlussstutzen (3) an seinem Außenumfang eine Hinterschneidung mit mindestens einer Nut (4) hat.

6. Medizinisches Gerät (100) mit einer Verbindungsleitung (103), die einerseits an dem medizinischen Gerät (100) befestigt ist, **dadurch gekennzeichnet, dass** an der Verbindungsleitung (103) andererseits eine Kupplung (102) befestigt ist, die zur lösbaren mechanischen und fluidischen Verbindung mit dem äußeren Anschlussstutzen (3) der Ansaugeinheit (6) gemäß einem der vorhergehenden Ansprüche ausgelegt und eingerichtet ist.

7. Medizinisches Gerät (100) nach Anspruch 6, **dadurch gekennzeichnet, dass** an einem Innenumfang der Kupplung (102) Rastkörper (120) angeordnet sind, die über eine axial bewegliche Betätigungshülse (110) radial in die Nut (4) bewegbar sind.

8. Medizinisches Gerät (100) nach Anspruch 6 oder 7, **dadurch gekennzeichnet, dass** an dem medizinischen Gerät (100) ein Stecknippel (104) angeordnet ist, auf den die Kupplung (102) steckbar ist.

9. Medizinisches Gerät (100) nach Anspruch 8, ferner mit einer Sensorik, die in Wirkverbindung mit dem Stecknippel (104) ist, und die zum Detektieren der aufgesteckten Kupplung (102) ausgelegt und eingerichtet ist.

10. Medizinisches Gerät (100) nach einem der Ansprüche 6 bis 9 mit einer Abstellfläche (101) für den Behälter (10).

11. Medizinische Anordnung, insbesondere Dialyseanordnung, mit einem medizinischen Gerät (100) gemäß einem der Ansprüche 6 bis 10 und mit einem Behälter (10), wobei das medizinische Gerät (100) und der Behälter (10) mittels einer Ansaugeinheit (6) gemäß einem der Ansprüche 1 bis 5 mechanisch und fluidisch miteinander lösbar verbunden sind.
